# EUROPÄISCHE PATENTSCHRIFT

(11) **EP 0 414 986 B1**
(45) Veröffentlichungstag und Bekanntmachung des Hinweises auf die Patenterteilung: **09.06.1993**
(21) Anmeldenummer: 90101762.4
(22) Anmeldetag: 30.01.1990
(51) Int. Cl.: A61F 9/02

(54) **Schutzbrille gegen Gase**
Goggle against gases
Lunette protectrice contre des gaz

(30) Priorität: 30.08.1989 DE 3928696
(43) Veröffentlichungstag der Anmeldung: 06.03.1991
(73) Patentinhaber: AUERGESELLSCHAFT GMBH, D-12059 Berlin (DE)
(72) Erfinder: Hünnebeck, Volker, D-1000 Berlin 45 (DE)

(56) Entgegenhaltungen:
- EP-A- 0 318 647
- CH-A- 490 849
- DE-A- 1 516 460
- DE-A- 3 307 200
- FR-A- 920 668
- GB-A- 179 461
- NL-A- 46 444
- US-A- 1 404 039

## Beschreibung

Die Erfindung bezieht sich auf eine Schutzbrille gegen Gase, gemäß dem Oberbegriff des Anspruchs 1.

Bei einer bekannten Schutzbrille der gattungsgemäßen Art sind die Eassungen der Augengläser jeweils als ein die Augengläser beidseitig umfassender Rahmen ausgebildet, der einen vorderen und einen hinteren Fassungsrand aufweist (EP-A- 318 647). Es ist weiterhin eine Schutzbrille bekannt, bei der die Augengläser jeweils auf starre, abgestufte Abschnitte gesetzt sind. Die Abschnitte sind an den Vorderrändern zylindrischer Körper ausgebildet und dort mittels eines Anschlag- und Halteringes befestigt (DE-A-33 07 200). Bei dieser Ausführung kann keine absolute Dichtheit zwischen den Augengläsern und den abgestuften Abschnitten erreicht werden.

Der Erfindung liegt die Aufgabe zugrunde, diesen Nachteil bei einer Schutzbrille der eingangs genannten Art zu beseinigen und eine Schutzbrille zu schaffen, bei der mit einfachen und wirkungsvollen Mitteln die Augengläser in den Fassungen sicher und dichtgehalten werden.

Diese Aufgabe wird durch die kennzeichnenden Merkmale im Anspruch 1 gelöst.

Eine vorteilhafte Weiterentwicklung ist in dem abhängigen Anspruch 2 gekennzeichnen.

Die mit der Erfindung erzielten Vorteile bestehen insbesondere darin, daß ohne dem Vorsehen von Spann- bzw. Schraubelementen eine sichere Dichtungs- und Spannmöglichkeit für die Augengläser in den Fassungen gewährleistet wird.

Ein Ausführungsbeispiel der Erfindung ist in der Zeichnung dargestellt und wird im folgenden näher beschrieben.
Es zeigt
- Fig. 1: eine Draufsicht der Schutzbrille, teils Ansicht und teils geschnitten, und
- Fig. 2: eine vergrößerte Schnittdarstellung der erfindungsgemäßen Fassung mit Augengläsern.

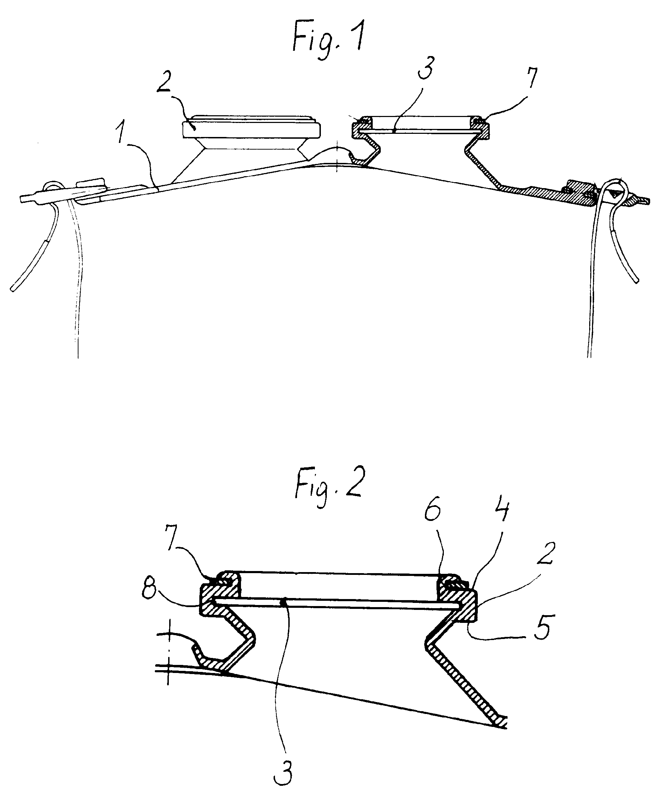

Wie aus den Fig. 1 und 2 ersichtlich ist, besteht die Schutzbrille im wesentlichen aus einem Brillenkörper 1 mit zwei Fassungen 2 zur Aufnahme von Augengläsern 3. Die Fassungen 2 sind jeweils als ein die Augengläser 3 außen umfassender Rahmen ausgebildet, der einen vorderen Rand 4 und einen hinteren Rand 5 sowie eine Vertiefung 8 zur Aufnahme des Augenglases 3 aufweist. Auf der Fläche des vorderen Randes 4 ist eine ringsum verlaufende Nutausführung 6 angeordnet, in die ein Ring 7 festgelegt wird. Der Ring 7 ist vorteilhaft als eine flache Scheibe ausgebildet, die in die Nut 6 eingeknöpft wird, wobei die Scheibe unter einer Vorspannung den vorderen Rand 4 der Fassung 2 gegen das in die Vertiefung 8 eingesetzte Augenglas 3 gleichmäßig andrückt. Durch diese Maßnahme werden die Augengläser 3 in der Fassung 2 unverlierbar fixiert und zusätzlich nach außen hin abgedichtet.

## Patentansprüche

1. Schutzbrille gegen Gase, bestehend aus einem elastischen Brillenkörper (1) mit zwei, jeweils ein Auge des Benutzers umschließenden Augenräumen, wobei der Brillenkörper (1) zur Aufnahme von Augengläsern (3) Fassungen (2) bildet, die jeweils als ein die Augengläser (3) außen umfassender Rahmen ausgebildet sind, der einen vorderen, den Außenteil des Augenglases teilweise abdeckenden Abschnitt mit der Außenfläche (4) und einen hinteren, den Innenteil des Augenglases teilweise abdeckenden Abschnitt mit der Außenfläche (5) sowie eine Radialvertiefung (8) zur Aufnahme des Augenglases (3) aufweist, dadurch gekennzeichnet, daß ein Ring (7) auf der Außenfläche (4) des vorderen Abschnitts des Rahmens liegend in einer ringsum verlaufenden, radialen Nut (6) mit Vorspannung eingelassen ist, in der Weise, daß der Ring (7) den zwischen ihm und dem Augenglas (3) befindlichen Abschnitt gleichmäßig gegen das Augenglas (3) andrückt.

2. Schutzbrille nach Anspruch 1, dadurch gekennzeichnet, daß der Ring (7) als eine flache Scheibe ausgebildet ist.

## Claims

1. Protective goggles against gases, consisting of an elastic goggles body (1) with two eye frames, in each case surrounding one eye of the user, in which the goggles body (1) forms mountings (2) to receive eye glasses (3), which in each case are constructed as a frame surrounding the eye glasses (3) externally, which frame has a front section, partially covering the external part of the eye glass, with the outer surface (4), and a rear section, partially covering the inner part of the eye glass, with the outer surface (5), and also has a radial depression (8) to receive the eye glass (3), characterised in that a ring (7), lying on the outer surface (4) of the front section of the frame, is let into a radial groove (6), with pre-stressing, running around it, such that the ring (7) uniformly presses the section situated between it and the eye glass (3) against the eye glass (3).

2. Protective goggles according to Claim 1, characterised in that the ring (7) is constructed as a flat disc.

## Revendications

1. Lunette de protection contre des gaz, constituée par un corps élastique de lunette (1) comportant deux espaces pour les yeux, qui entourent chacun un oeil de l'utilisateur, et dans laquelle le corps (1) de la lunette forme, pour y loger des verres (3), des montures (2), qui sont agencées chacune sous la forme d'un cadre entourant extérieurement le verre (3) et qui possède une section avant, qui recouvre partiellement la partie extérieure du verre et forme la surface extérieure (4), et une section arrière, qui recouvre partiellement la partie intérieure du verre et possède la surface extérieure (5), ainsi qu'un renfoncement radial (8) servant à loger le verre (3), caractérisée en ce qu'une bague (7) est insérée sous précontrainte dans une rainure radiale circonférentielle (6), en s'appliquant contre la surface extérieure (4) de la section avant du cadre, de sorte que la bague (7) serre uniformément contre la section située entre l'anneau et le verre (3), contre ce dernier.

2. Lunette de protection suivant la revendication 1, caractérisée en ce que la bague (7) est réalisée sous la forme d'une rondelle plate.
